# EUROPEAN PATENT SPECIFICATION

(11) **EP 3 448 426 B1**
(45) Date of publication and mention of the grant of the patent: **28.05.2025**
(21) Application number: 17722604.0
(22) Date of filing: 27.04.2017
(51) Int. Cl.: A61K 31/404, A61K 31/47, A61K 31/366, A61K 31/505, A61K 45/06, A61P 3/06, A61K 31/22, A61K 31/40, A61K 31/4468, A61K 31/397, A61K 9/00, A61P 9/10, A61P 43/00, A61K 39/395, C07K 16/22

(54) **METHODS FOR TREATING PATIENTS WITH FAMILIAL HYPERCHOLESTEROLEMIA**
VERFAHREN ZUR BEHANDLUNG VON PATIENTEN MIT FAMILIÄRER HYPERCHOLESTERINÄMIE
MÉTHODES DE TRAITEMENT DE PATIENTS PRÉSENTANT UNE HYPERCHOLESTÉROLÉMIE FAMILIALE

(30) Priority: 28.04.2016 US 201662328823 P; 09.06.2016 US 201662348001 P; 27.01.2017 US 201762451310 P
(43) Date of publication of application: 06.03.2019
(73) Proprietor: Regeneron Pharmaceuticals, Inc., Tarrytown, NY 10591 (US)
(72) Inventor: PORDY, Robert C., Tarrytown, New York 10591 (US); SASIELA, William J., Tarrytown, New York 10591 (US); SCHWEMMER-GIPE, Daniel A., Tarrytown, New York 10591 (US)
(74) Representative: J A Kemp LLP
(86) International application number: PCT/US2017/029782
(87) International publication number: WO 2017/189813

(56) References cited:
- EP-B1- 3 439 689
- WO-A1-2012/174178
- WO-A1-2015/100394
- WO-A1-2017/151783
- WO-A2-2008/073300
- WO-A2-2011/085271
- US-A1- 2013 064 834
- HARTGERS MEREL L ET AL: "New Approaches in Detection and Treatment of Familial Hypercholesterolemia", CURRENT CARDIOLOGY REPORTS, CURRENT SCIENCE, PHILADELPHIA, PA, US, vol. 17, no. 12, 19 October 2015 (2015-10-19), pages 1 - 8, XP035572123, ISSN: 1523-3782, [retrieved on 20151019], DOI: 10.1007/S11886-015-0665-X
- GAUDET DANIEL ET AL: "Safety and efficacy of evinacumab, a monoclonal antibody to ANGPTL3, in patients with homozygous familial hypercholesterolemia receiving concomitant lipid-lowering therapies", JOURNAL OF CLINICAL LIPIDOLOGY, vol. 10, no. 3, 17 May 2016 (2016-05-17) - 17 May 2016 (2016-05-17), pages 715, XP029538107, ISSN: 1933-2874, DOI: 10.1016/J.JACL.2016.03.091
- YAN WANG ET AL: "Inactivation of ANGPTL3 reduces hepatic VLDL-triglyceride secretion", JOURNAL OF LIPID RESEARCH, vol. 56, no. 7, 1 July 2015 (2015-07-01), US, pages 1296 - 1307, XP055385805, ISSN: 0022-2275, DOI: 10.1194/jlr.M054882
- VIKTORIA GUSAROVA ET AL: "ANGPTL3 blockade with a human monoclonal antibody reduces plasma lipids in dyslipidemic mice and monkeys", JOURNAL OF LIPID RESEARCH, vol. 56, no. 7, 29 July 2015 (2015-07-29), US, pages 1308 - 1317, XP055363055, ISSN: 0022-2275, DOI: 10.1194/jlr.M054890
- GAUDET DANIEL ET AL: "Safety and Efficacy of Evinacumab, A Monoclonal Antibody to ANGPTL3, In Homozygous Familial Hypercholesterolemia", JOURNAL OF CLINICAL LIPIDOLOGY, vol. 11, no. 3, May 2017 (2017-05-01) - May 2017 (2017-05-01), pages 837 - 838, XP085021299, ISSN: 1933-2874, DOI: 10.1016/J.JACL.2017.04.106

## Description

### FIELD OF THE INVENTION

The present invention relates to the field of therapeutic treatments of diseases and disorders, which are associated with elevated levels of lipids and lipoproteins. More specifically, the invention relates to the use of an ANGPTL3 inhibitor with concomitant lipid-lowering therapies to treat patients with familial hypercholesterolemia in order to achieve optimal serum lipid and lipoprotein levels.

### BACKGROUND

Hyperlipidemia is a general term that encompasses diseases and disorders characterized by or associated with elevated levels of lipids and/or lipoproteins in the blood. Hyperlipidemias include hypercholesterolemia, hypertriglyceridemia, combined hyperlipidemia, and elevated lipoprotein a (Lp(a)). A particular prevalent form of hyperlipidemia in many populations is hypercholesterolemia.

Hypercholesterolemia, particularly an increase in low-density lipoprotein (LDL) cholesterol (LDL-C) levels, constitutes a major risk for the development of atherosclerosis and coronary heart disease (CHD) (Sharrett et al., 2001, Circulation 104:1108-1113). Low-density lipoprotein cholesterol is identified as the primary target of cholesterol lowering therapy and is accepted as a valid surrogate therapeutic endpoint. Numerous studies have demonstrated that reducing LDL-C levels reduces the risk of CHD with a strong direct relationship between LDL-C levels and CHD events; for each 1 mmol/L (~40 mg/dL) reduction in LDL-C, cardiovascular disease (CVD) mortality and morbidity is lowered by 22%. Greater reductions in LDL-C produce greater reduction in events, and comparative data of intensive versus standard statin treatment suggest that the lower the LDL-C level, the greater the benefit in patients at very high cardiovascular (CV) risk.

Familial hypercholesterolemia (FH) is an inherited disorder of lipid metabolism that predisposes a person to premature severe cardiovascular disease (CVD) (Kolansky et al., (2008), Am J Cardiology,102(11):1438-1443). FH can be either an autosomal dominant or an autosomal recessive disease that results from mutations in the low density lipoprotein receptor (LDLR), or in at least 3 different genes that code for proteins involved in hepatic clearance of LDL-C can cause FH. Examples of such defects include mutations in the gene coding for the LDL receptor (LDLR) that removes LDL-C from the circulation, and in the gene for apolipoprotein (Apo) B, which is the major protein of the LDL particle. In all cases, FH is characterized by an accumulation of LDL-C in the plasma from birth and subsequent development of tendon xanthomas, xanthelasmas, atheromata, and CVD. FH can be classified as either heterozygous FH (heFH) or homozygous FH (hoFH) depending on whether the individual has a genetic defect in one (heterozygous) or both (homozygous) copies of the implicated gene.

Current LDL-C-lowering medications include statins, cholesterol absorption inhibitors, fibrates, niacin, bile acid sequestrants and Proprotein Convertase Subtilisin/Kexin Type 9 (PCSK9) inhibitors. Statins are a commonly prescribed treatment for LDL-C lowering. However, despite the availability of such lipid-lowering therapies, many high-risk patients fail to reach their guideline target LDL-C level (Gitt et al., 2010, Clin Res Cardiol 99(11):723-733). For patients who are still unable to achieve guideline target level for LDL-C, despite available lipid-modifying therapy (LMT), mechanical removal of LDL-C by lipoprotein apheresis (e.g., LDL apheresis) is sometimes prescribed.

Hartgers et al (2015) Curr Cardiol Rep, 17, 109 discloses approaches in the detection and treatment of familial hypercholesterolemia. EP 3439689 discloses methods of treating hyperlipidemia using an ANGPTL8 inhibitor and an ANGPTL3 inhibitor. WO 2017/151783 discloses methods of treating hyperlipidemia using a PCSK9 inhibitor in combination with an ANGPTL3 inhibitor.

Patients who are not at LDL-C goal despite receiving an optimized LMT regimen, would greatly benefit from alternative LDL-C lowering therapies, or through use of a combination of therapeutic agents, such as the agents and regimens described herein.

### BRIEF SUMMARY OF THE INVENTION

The invention relates to an ANGPTL3 inhibitor for use in methods of treating patients who suffer from familial hypercholesterolemia in combination with other lipid modifying therapies to achieve optimal levels of serum lipids and lipoproteins.

Specifically, the invention provides an inhibitor of ANGPTL3 for use in a method of treating a patient suffering from familial hypercholesterolemia in combination with a statin and two lipid-lowering agents other than a statin, wherein the method comprises administering to the patient a therapeutically effective amount of the statin, the two lipid-lowering agents other than a statin and an inhibitor of ANGPTL3, wherein
the ANGPTL3 inhibitor is evinacumab;
the statin is selected from the group consisting of atorvastatin, pitavastatin, lovastatin, simvastatin, pravastatin, fluvastatin and rosuvastatin; and
the first lipid-lowering agent other than a statin is an agent that inhibits cholesterol absorption

In one embodiment, the familial hypercholesterolemia is selected from the group consisting of heterozygous familial hypercholesterolemia (HeFH) and homozygous familial hypercholesterolemia (HoFH).

In one embodiment, the statin is rosuvastatin, which is administered orally once a day at a dose of about 5 mg to about 40 mg. In another embodiment, the statin is rosuvastatin, which is administered orally once a day at a dose of 5-40 mg.

In one embodiment, the statin is atorvastatin, which is administered orally once a day at a dose of about 10 mg to about 80 mg. In another embodiment, the statin is atorvastatin, which is administered orally once a day at a dose of 10-80 mg.

In one embodiment, the agent that inhibits cholesterol absorption is ezetimibe.

In one embodiment, the ezetimibe is administered orally once a day at a dose of about 10 mg. In another embodiment, the ezetimibe is administered orally once a day at a dose of 10 mg.

In one embodiment, the second lipid lowering agent other than a statin is an agent that inhibits microsomal triglyceride transfer protein (MTTP).

In one embodiment, the agent that inhibits microsomal triglyceride transfer protein is lomitapide.

In one embodiment, the lomitapide is administered orally once a day at a dose of about 5 mg to about 60 mg. In another embodiment, the lomitapide is administered orally once a day at a dose of 5-60 mg.

In one embodiment, the lomitapide is administered orally once a day at a dose of about 20 mg. In another embodiment, the lomitapide is administered orally once a day at a dose of 20 mg.

In one embodiment, evinacumab is administered before, during, or after treatment with a statin, ezetimibe, lomitapide, mipomersen, a PCSK9 inhibitor, or any other lipid lowering agent established to be useful for achieving normalization of at least one lipid parameter described herein.

In one embodiment, evinacumab is administered intravenously at a dose ranging from about 1 mg/kg to about 20 mg/kg of body weight.

In one embodiment, evinacumab is administered intravenously at a dose of about 15 mg/kg of body weight. In another embodiment, evinacumab is administered intravenously at a dose of 15 mg/kg of body weight.

In one embodiment, evinacumab is administered subcutaneously at a dose ranging from about 50 mg to about 750 mg.

In one embodiment, evinacumab is administered subcutaneously at a dose ranging from about 250 mg to about 450 mg.

In one embodiment, evinacumab is administered every week, every two weeks, every 3 weeks, every 4 weeks, every 2 months, every 3 months, or every 4 months.

### DETAILED DESCRIPTION

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. As used herein, the term "about," when used in reference to a particular recited numerical value, means that the value may vary from the recited value by no more than 1%. For example, as used herein, the expression "about 100" includes 99 and 101 and all values in between (e.g., 99.1, 99.2, 99.3, 99.4, etc.).

Although any methods and materials similar or equivalent to those described herein can be used in the practice of the present invention, the preferred methods and materials are now described.

### Methods for Treating Hyperlipidemias

The present invention relates to an inhibitor of ANGPTL3 for use in reducing lipoprotein levels in patients suffering from familial hypercholesterolemia, by administering a combination of the ANGPTL3 inhibitor and (a) a statin; (b) a first lipid lowering therapy other than a statin; and (c) a second lipid lowering agent other than a statin. The first lipid-lowering agent that is not a statin is an agent that inhibits cholesterol absorption, such as ezetimibe (ZETIA^{®}). In certain embodiments, the second lipid lowering agent that is not a statin is an agent that inhibits microsomal triglyceride transfer protein, such as lomitapide (JUSTAPID^{®}). The ANGPTL3 inhibitor is an antibody that binds specifically to ANGPTL3, specifically evinacumab. In certain embodiments of the invention, treatment with the combination of an ANGPTL3 inhibitor (evinacumab) with the other therapies noted above (a statin, ezetimibe and lomitapide), may serve to lower the levels of lipoproteins in these patients to an acceptable range, thereby lowering their risk for development of atherosclerosis, stroke and other cardiovascular diseases. In certain embodiments, the methods described may be used to treat patients suffering from familial hypercholesterolemia, including heterozygous familial hypercholesterolemia (HeFH) and/or homozygous familial hypercholesterolemia (HoFH). In certain embodiments, a PCSK9 inhibitor may also be added to the combined therapies described above to further lower the level of at least one lipid parameter described herein. In a related embodiment, the combination of therapies described above may also be used in patients that are undergoing apheresis to achieve normalization of at least one of the lipid parameters described. The combination of therapies described may eliminate the need for apheresis, or may increase the time interval between the need for apheresis procedures. The combination of therapies described, when used alone or in combination with apheresis may serve to lower the risk for the development of atherosclerosis and coronary heart disease (CHD) in these patients.

As used herein, the term "lipoprotein" means a biomolecular particle containing both protein and lipid. Examples of lipoproteins include, e.g., low density lipoprotein (LDL), high-density lipoprotein (HDL), very low density lipoprotein (VLDL), intermediate density lipoprotein (IDL), and lipoprotein (a) (Lp(a)).

The present invention, according to certain embodiments, includes treating patients who are non-responsive to, inadequately controlled by, or intolerant to lipid modifying therapies, other than those described and included in the combination described herein. As used herein, a particular patient who is "non-responsive to, inadequately controlled by, or intolerant to, lipid modifying therapy" is determined by a physician, physician's assistant, diagnostician, or other medical professional on the basis of the level of one or more lipoproteins (*e*.*g*., LDL-C and/or non-HDL-C) measured or otherwise detected in the serum of the patient after treatment with the lipid modifying agent. The physician, physician's assistant, diagnostician, or other medical professional can also determine if the patient is intolerant to certain lipid modifying therapies based on the side effect profile of the lipid modifying therapies, which the patient may experience, including, but not limited to, muscle aches, tenderness or weakness (myalgia), headache, skin flushing, difficulty sleeping, abdominal cramping, bloating, diarrhea, constipation, rash, nausea, or vomiting. A patient who is non-responsive to, inadequately controlled by, or intolerant to certain lipid modifying therapy may also be determined or influenced by other factors such as the patient's family history, medical background, current therapeutic treatment status, as well as generally accepted or prevailing lipoprotein targets adopted by national medical associations and physicians' groups. For example, in certain contexts, if a patient is undergoing therapy with a certain lipid modifying agent, and exhibits an LDL-C level of greater than or equal to about 70 mg/dL, this indicates that the patient is "non-responsive to, or inadequately controlled by, or intolerant to that lipid modifying therapy" and may benefit by treatment using the therapies described herein. In other contexts, if a patient is undergoing therapy with a certain lipid modifying agent, and exhibits an LDL-C level of greater than or equal to about 100 mg/dL, this indicates that the patient is "non-responsive to, inadequately controlled by, or intolerant to that lipid modifying therapy" and may benefit by treatment using the therapies described herein. In certain contexts, if a patient is undergoing therapy with a certain lipid modifying agent, and exhibits an LDL-C level of greater than or equal to about 150 mg/dL, 200 mg/dL, 250 mg/dL, 300 mg/dL, 400 mg/dL or higher, this indicates that the patient is "non-responsive to, inadequately controlled by, or intolerant to a certain lipid modifying therapy" and may benefit by treatment using the therapies described herein. In yet other contexts, whether or not a particular percentage reduction in LDL-C or non-HDL-C level is met, relative to the patient's LDL-C or non-HDL-C level at a particular start point ("baseline") can be used to determine whether the patient has responded to a lipid modifying therapy or whether that patient is in need of further treatment using the present invention. For instance, a reduction in LDL-C or non-HDL-C of less than 50% (*e*.*g*., less than 40%, less than 35%, less than 30%, less than 25%, etc.) from baseline may signify a need for therapy using the invention.

The present invention, accordingly, includes administering one or more doses of an ANGPTL3 inhibitor (evinacumab) and one or more doses of a combination of a statin, ezetimibe, a PCSK9 inhibitor, mipomersen and/or lomitapide to a patient who is undergoing other types of lipid modifying therapy (e.g. bile acid sequestrants, niacin, fenofibrate), but is non-responsive to such therapy, or is intolerant to such therapy, wherein, after receiving one or more doses of the combination therapy described herein, the patient is able to achieve normal levels of total cholesterol, LDL-C, or non-HDL-C. In certain instances, the patient may be taken off of the other lipid modifying therapy, or the other lipid modifying therapy may be continued, but may be administered at lower doses and may be used in combination with the ANGPTL3 inhibitor and a statin plus ezetimibe and lomitapide, and optionally, a PCSK9 inhibitor, and/or mipomersen to achieve and/or maintain a particular target lipoprotein level. Alternatively, the patient may be administered the other lipid modifying therapy at the normal prescribed dose, but the frequency of administration of the other lipid modifying therapy may be reduced if the other lipid modifying therapy is to be administered in conjunction with the combination described herein. In some instances, the need for treatment with the other lipid modifying therapy by the patient to achieve and/or maintain a particular target lipoprotein level may be eliminated altogether following administration of one or more doses of the combination therapies described herein.

According to certain embodiments, the present invention comprises reducing or eliminating the need for certain lipid modifying therapy, wherein the methods comprise selecting a patient with hyperlipidemia (*e*.*g*., hypercholesterolemia) who has been treated with certain lipid modifying therapies within the last month, the last 2 months, the last 3 months, the last 4 months, the last 5 months, the last 6 months, or for a longer period, and administering one or more doses of an ANGPTL3 inhibitor in combination with the agents described herein (ezetimibe, lomitapide and a statin) to the patient. The methods according to this aspect of the invention result in lowering the level of at least one lipid, or lipoprotein in the serum of the patient, and consequently allow for a reduction or elimination of the need for treatment with the other lipid modifying therapy to which the patient did not respond (e.g. a bile acid sequestrant, niacin, or fenofibrate), or for which the patient showed an intolerance. The methods described herein may also be used in patients undergoing apheresis and the combination of lipid lowering agents used in this patient population may result in elimination of the need for apheresis, or may increase the time interval between apheresis procedures. For example, in certain embodiments of the present invention, following administration of one or more doses of an ANGPLT3 inhibitor in combination with a statin, ezetimibe and/or lomitapide, the serum LDL-C level of the patient is reduced to less than a defined level (*e*.*g*., less than 100 mg/dL or less than 70 mg/dL), or the total cholesterol is lowered to a defined level (*e*.*g*. less than 200 mg/dL, or less than 150 mg/dL, or the serum level of LDL-C shows at least a 40% reduction compared to the baseline levels before treatment with the combination described herein.

According to certain embodiments, the patient who is treatable in the present invention has hypercholesterolemia (*e*.*g*., a serum LDL-C concentration of greater than or equal to 70 mg/dL (*e*.*g*. if the patient has a history of a cardiovascular event), or a serum LDL-C concentration greater than or equal to 100 mg/dL (*e*.*g*. if the patient has no history of a cardiovascular event). In certain embodiments, the patient's hypercholesterolemia is inadequately controlled by certain standard lipid modifying therapies, such as bile acid sequestrants, niacin, or fenofibrates. The present invention may also reduce total cholesterol, LDL-C, non-HDL-C, triglycerides (TG), ApoB, ApoClll and Lp(a) in a patient who has familial hypercholesterolemia, including HeFH and HoFH.

### Patients Suitable for Treatment

The present disclosure includes methods and compositions useful for treating patients who are diagnosed with or identified as being at risk of developing a hypercholesterolemia condition such as, *e*.*g*., Heterozygous Familial Hypercholesterolemia (HeFH) or Homozygous Familial Hypercholesterolemia (HoFH) resulting from mutations in the low-density lipoprotein receptor (LDLR), Autosomal Dominant Hypercholesterolemia (ADH, *e*.*g*., ADH associated with one or more gain-of-function mutations in the PCSK9 gene), documented presence of homozygous or compound heterozygous mutations in the Apo B gene, autosomal recessive hypercholesterolemia (ARH, *e*.*g*., ARH associated with mutations in LDLRAP1), as well as incidences of hypercholesterolemia that are distinct from Familial Hypercholesterolemia (non FH). In the invention, the patient is suffering from familial hypercholesterolemia. A patient who is suitable for treatment may also include patients who exhibit LDLR mutations that fall within any of the following classes: Class I: Receptor null mutations, whereby LDLR is not synthesized at all; Class II: Transport defective alleles, whereby LDLR is not properly transported from the endoplasmic reticulum to the Golgi apparatus for expression on the cell surface (class IIA (no receptor transport) and class IIB (reduced receptor transport); Class III: Binding defective alleles, whereby LDLR does not properly bind LDL on the cell surface because of a defect in either apolipoprotein B100 (R3500Q) or in LDL-R; Class IV: Internalization defective alleles whereby LDLR bound to LDL does not properly cluster in clathrin-coated pits for receptor-mediated endocytosis; Class V: Recycling defective alleles, whereby LDLR is not recycled back to the cell surface.

Diagnosis of familial hypercholesterolemia (*e*.*g*., heFH or hoFH) can be made by genotyping and/or clinical criteria. For patients who are not genotyped, clinical diagnosis may be based on either the Simon Broome criteria with a criteria for definite FH, or the WHO/Dutch Lipid Network criteria with a score > 8 points.

According to certain embodiments, a patient may be suitable for treatment on the basis of having a history of coronary heart disease (CHD). As used herein a "history of CHD" (or "documented history of CHD") includes one or more of: (i) acute myocardial infarction (MI); (ii) silent MI; (iii) unstable angina; (iv) coronary revascularization procedure (e.g., percutaneous coronary intervention [PCI] or coronary artery bypass graft surgery [CABG]); and/or (v) clinically significant CHD diagnosed by invasive or non-invasive testing (such as coronary angiography, stress test using treadmill, stress echocardiography or nuclear imaging).

According to certain embodiments, a patient may be suitable for treatment on the basis of having non-coronary heart disease cardiovascular disease ("non-CHD CVD"). As used herein, "non-CHD CVD" includes one or more of: (i) documented previous ischemic stroke with a focal ischemic neurological deficit that persisted more than 24 hours, considered as being of atherothrombotic origin; (ii) peripheral arterial disease; (iii) abdominal aortic aneurysm; (iv) atherosclerotic renal artery stenosis; and/or (v) carotid artery disease (transient ischemic attacks or >50% obstruction of a carotid artery).

According to certain embodiments, a patient may be suitable for treatment on the basis of having one or more additional risk factors such as, *e*.*g*., (i) documented moderate chronic kidney disease (CKD) as defined by 30 ≤eGFR <60 mL/min/1.73 m2 for 3 months or more; (ii) type 1 or type 2 diabetes mellitus with or without target organ damage (*e*.*g*., retinopathy, nephropathy, microalbuminuria); (iii) a calculated 10-year fatal CVD risk SCORE ≥5% (ESC/EAS Guidelines for the management of dyslipidemias, Conroy et al., 2003, Eur. Heart J. 24:987-1003).

According to certain embodiments, a patient may be suitable for treatment on the basis of having one or more additional risk factors selected from the group consisting of age (*e*.*g*., older than 40, 45, 50, 55, 60, 65, 70, 75, or 80 years), race, national origin, gender (male or female), exercise habits (*e*.*g*., regular exerciser, non-exerciser), other preexisting medical conditions (*e.g.,* type-II diabetes, high blood pressure, etc.), and current medication status (*e.g.,* currently taking beta blockers, niacin, ezetimibe, fibrates, omega-3 fatty acids, bile acid resins, etc.).

According to certain embodiments of the present invention, a subject who is treatable may exhibit an elevated level of one or more inflammatory marker. Any marker of systemic inflammation can be utilized for the purposes of the present invention. Suitable inflammatory markers include, without limitation, C-reactive protein, cytokines (*e.g*., II-6, IL-8, and/or IL-17), and cellular adhesion molecules (*e*.*g*., ICAM-1, ICAM-3, BL-CAM, LFA-2, VCAM-1, NCAM, and PECAM).

According to the present invention, patients may be suitable for treatment on the basis of a combination of one or more of the foregoing criteria or therapeutic characteristics. For example, according to certain embodiments, a patient suitable for treatment may further be selected on the basis of having HeFH in combination with: (i) a history of documented CHD, (ii) non-CHD CVD, and/or (iii) diabetes mellitus with target organ damage; such patients may also be selected on the basis of having a serum LDL-C concentration of greater than or equal to 70 mg/dL.

According to certain other embodiments, a patient suitable for treatment, in addition to having hypercholesterolemia that is not adequately controlled by a daily moderate-dose therapeutic statin regimen, may further be selected on the basis of having HeFH without CHD, or non-CHD CVD, but having either (i) a calculated 10-year fatal CVD risk SCORE ≥5%; or (ii) diabetes mellitus without target organ damage; such patients may also be selected on the basis of having a serum LDL-C concentration of greater than or equal to 100 mg/dL.

According to certain aspects, the subject who is treatable by the methods disclosed herein is a subject who has familial chylomicronemia syndrome (FCS; also known as lipoprotein lipase deficiency).

According to certain embodiments of the present invention, the subject who is treatable is a subject who is undergoing, or has recently undergone, lipoprotein apheresis (e.g., within the last six months, within the last 12 weeks, within the last 8 weeks, within the last 6 weeks, within the last 4 weeks, within the last 2 weeks, etc.).

### Administration of an ANGPTL3 Inhibitor as Add-On Therapy

The present disclosure includes methods of treatment wherein a patient who is undergoing, or has recently undergone, standard lipid modifying therapy (*e*.*g*. a statin) is administered an ANGPTL3 inhibitor according to a particular dosing amount and frequency, and wherein the ANGPTL3 inhibitor is administered as an add-on to the patient's pre-existing lipid modifying therapy (if applicable), such as an add-on to the patient's pre-existing daily therapeutic statin regimen, or other regimen, e.g. niacin. The invention includes use of the ANGPTL3 inhibitor (evinacumab) as add on therapy with lipid modifying therapies in addition to statins, including use with ezetimibe and lomitapide to achieve maximal lipid lowering effects. Additional lipid lowering agents to be used in the invention include PCSK9 inhibitors, or mipomersen. The combination of agents may also be used in patients undergoing apheresis to achieve acceptable lipid levels.

For example, the methods of the present disclosure include add-on therapeutic regimens wherein the ANGPTL3 inhibitor is administered as add-on therapy to the same stable daily therapeutic statin regimen *(i.e.,* same dosing amount of statin) that the patient was on prior to receiving the ANGPTL3 inhibitor. In addition to the statin plus the ANGPTL3 antibody therapy, the addition of either ezetimibe alone, or in combination with lomitapide results in significantly lower levels of serum lipids or lipoproteins when the combination is administered. In other aspects, the ANGPTL3 inhibitors are administered as add-on therapy to a therapeutic statin regimen comprising a statin in an amount that is more than or less than the dose of statin the patient was on prior to receiving the ANGPTL3 inhibitor, or the combination therapy described herein. For example, after starting a therapeutic regimen comprising an ANGPTL3 inhibitor administered at particular dosing frequencies and amounts, in addition to ezetimibe and lomitapide, the daily dose of statin administered or prescribed to the patient may (a) stay the same, (b) increase, or (c) decrease (*e*.*g*., up-titrate or down-titrate) in comparison to the daily statin dose the patient was taking before starting the ANGPTL3 inhibitor, ezetimibe and/or lomitapide therapeutic regimen, depending on the therapeutic needs of the patient.

### Therapeutic Efficacy

The present invention may result in the reduction in serum levels of one or more lipid components selected from the group consisting of total cholesterol, LDL-C, IDL, non-HDL-C, ApoB 100, ApoB 48, Apo A-1, Apo CIII, VLDL-C, triglycerides, Lp(a), chylomicrons, chylomicron remnants, and remnant cholesterol. For example, according to certain embodiments of the present invention, administration of an ANGPTL3 inhibitor in combination with a statin, ezetimibe and lomitapide to a suitable subject will result in a mean percent reduction from baseline in serum low density lipoprotein cholesterol (LDL-C) of at least about 25%, 30%, 40%, 50%, 60%, or greater; a mean percent reduction from baseline in ApoB of at least about 25%, 30%, 40%, 50%, 60%, or greater; a mean percent reduction from baseline in non-HDL-C of at least about 25%, 30%, 40%, 50%, 60%, or greater; a mean percent reduction from baseline in total cholesterol of at least about 10%, 15%, 20%, 25%, 30%, 35%, or greater; a mean percent reduction from baseline in VLDL-C of at least about 5%, 10%, 15%, 20%, 25%, 30%, or greater; a mean percent reduction from baseline in triglycerides of at least about 5%, 10%, 15%, 20%, 25%, 30%, 35% or greater; and/or a mean percent reduction from baseline in Lp(a) of at least about 5%, 10%, 15%, 20%, 25%, or greater.

### ANGPTL3 Inhibitors

The present invention comprises administering to a patient a therapeutic composition comprising evinacumab in combination with a statin, an inhibitor of cholesterol absorption (e.g. ezetimibe), and an agent that inhibits microsomal triglyceride transfer protein (e.g. lomitapide).

As used herein, an " ANGPTL3 inhibitor" is any agent, which binds to or interacts with human ANGPTL3 and inhibits the normal biological function of ANGPTL3 *in vitro* or *in vivo.* Non-limiting examples of categories of ANGPTL3 inhibitors include small molecule ANGPTL3 antagonists, nucleic acid-based inhibitors of ANGPTL3 expression or activity (*e*.*g*., siRNA or antisense), peptide-based molecules that specifically interact with ANGPTL3 (*e*.*g*., peptibodies), receptor molecules that specifically interact with ANGPTL3, ANGPTL3-binding scaffold molecules (*e*.*g*., DARPins, HEAT repeat proteins, ARM repeat proteins, tetratricopeptide repeat proteins, fibronectin-based scaffold constructs, and other scaffolds based on naturally occurring repeat proteins, etc., [*see, e.g.,* Boersma and Pluckthun, 2011, Curr. Opin. Biotechnol. 22:849-857, and references cited therein]), and anti-ANGPTL3 aptamers or portions thereof. According to certain aspects of the disclosure, ANGPTL3 inhibitors that can be used are anti-ANGPTL3 antibodies or antigen-binding fragments of antibodies that specifically bind human ANGPTL3.

The term "human angiopoietin-like protein-3" or "human ANGPTL3" or "hANGPTL3", as used herein, refers to ANGPTL3 having the amino acid sequence of SEQ ID NO: 9 (see also NCBI Accession NP_055310), or a biologically active fragment thereof.

The term "antibody", as used herein, is intended to refer to immunoglobulin molecules comprising four polypeptide chains, two heavy (H) chains and two light (L) chains interconnected by disulfide bonds, as well as multimers thereof (*e*.*g*., IgM). Each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (C_{L}1). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different aspects of the disclosure, the FRs of the anti-ANGPTL3 antibody (or antigen-binding portion thereof) may be identical to the human germline sequences, or may be naturally or artificially modified. An amino acid consensus sequence may be defined based on a side-by-side analysis of two or more CDRs.

The term "antibody," as used herein, also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, *e*.*g*., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, *e*.*g*., commercial sources, DNA libraries (including, *e*.*g*., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (*e*.*g*., an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (*e*.*g*. monovalent nanobodies, bivalent nanobodies, etc.), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR, which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric V_{H} or V_{L} domain.

In certain aspects of the disclosure, an antigen-binding fragment of an antibody may contain at least one variable domain covalently linked to at least one constant domain. Non-limiting, exemplary configurations of variable and constant domains that may be found within an antigen-binding fragment of an antibody include: (i) V_{H}-C_{H}1; (ii) V_{H}-C_{H}2; (iii) V_{H}-C_{H}3; (iv) V_{H}-C_{H}1-C_{H}2; (v) V_{H}-C_{H}1-C_{H}2-C_{H}3; (vi) V_{H}-C_{H}2-C_{H}3; (vii) V_{H}-C_{L}; (viii) V_{L}-C_{H}1; (ix) V_{L}-C_{H}2; (x) V_{L}-C_{H}3; (xi) V_{L}-C_{H}1-C_{H}2; (xii) V_{L}-C_{H}1-C_{H}2-C_{H}3; (xiii) V_{L}-C_{H}2-C_{H}3; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (*e*.*g*., 5, 10, 15, 20, 40, 60 or more) amino acids, which result in a flexible or semi-flexible linkage between adjacent variable and/or constant domains in a single polypeptide molecule. Moreover, an antigen-binding fragment of an antibody may comprise a homo-dimer or hetero-dimer (or other multimer) of any of the variable and constant domain configurations listed above in non-covalent association with one another and/or with one or more monomeric V_{H} or V_{L} domain (*e*.*g*., by disulfide bond(s)).

As with full antibody molecules, antigen-binding fragments may be monospecific or multispecific (*e*.*g*., bispecific). A multispecific antigen-binding fragment of an antibody will typically comprise at least two different variable domains, wherein each variable domain is capable of specifically binding to a separate antigen or to a different epitope on the same antigen. Any multispecific antibody format, including the exemplary bispecific antibody formats disclosed herein, may be adapted for use in the context of an antigen-binding fragment of an antibody using routine techniques available in the art.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody", as used herein, is intended to include antibodies having variable and constant regions derived from human germline immunoglobulin sequences. The human antibodies of the invention may nonetheless include amino acid residues not encoded by human germline immunoglobulin sequences (e.g., mutations introduced by random or site-specific mutagenesis *in vitro* or by somatic mutation *in vivo*), for example in the CDRs and in particular CDR3. However, the term "human antibody", as used herein, is not intended to include antibodies in which CDR sequences derived from the germline of another mammalian species, such as a mouse, have been grafted onto human framework sequences. The term includes antibodies recombinantly produced in a non-human mammal, or in cells of a non-human mammal. The term is not intended to include antibodies isolated from or generated in a human subject.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain aspects of the disclosure, however, such recombinant human antibodies are subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

Human antibodies can exist in two forms that are associated with hinge heterogeneity. In one form, an immunoglobulin molecule comprises a stable four chain construct of approximately 150-160 kDa in which the dimers are held together by an interchain heavy chain disulfide bond. In a second form, the dimers are not linked via inter-chain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al. (1993) Molecular Immunology 30:105) to levels typically observed using a human IgG1 hinge. The instant disclosure encompasses antibodies having one or more mutations in the hinge, C_{H}2 or C_{H}3 region which may be desirable, for example, in production, to improve the yield of the desired antibody form.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody" for purposes of the present invention. An isolated antibody also includes an antibody *in situ* within a recombinant cell. Isolated antibodies are antibodies that have been subjected to at least one purification or isolation step. According to certain embodiments, an isolated antibody may be substantially free of other cellular material and/or chemicals.

The term "specifically binds," or the like, means that an antibody or antigen-binding fragment thereof forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" ANGPTL3, as used in the context of the present disclosure, includes antibodies that bind ANGPTL3, or a portion thereof with a K_{D} of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or less than about 0.5 nM, as measured in a surface plasmon resonance assay. An isolated antibody that specifically binds human ANGPTL3, however, has cross-reactivity to other antigens, such as ANGPTL3 molecules from other (non-human) species.

The anti-ANGPTL3 antibodies useful for the methods of the present disclosure may comprise one or more amino acid substitutions, insertions and/or deletions in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases. The present disclosure includes methods involving the use of antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations"). A person of ordinary skill in the art, starting with the heavy and light chain variable region sequences disclosed herein, can easily produce numerous antibodies and antigen-binding fragments which comprise one or more individual germline mutations or combinations thereof. In certain aspects of the disclosure, all of the framework and/or CDR residues within the V_{H} and/or V_{L} domains are mutated back to the residues found in the original germline sequence from which the antibody was derived. In other aspects, only certain residues are mutated back to the original germline sequence, *e*.*g*., only the mutated residues found within the first 8 amino acids of FR1 or within the last 8 amino acids of FR4, or only the mutated residues found within CDR1, CDR2 or CDR3. In other aspects, one or more of the framework and/or CDR residue(s) are mutated to the corresponding residue(s) of a different germline sequence *(i.e.,* a germline sequence that is different from the germline sequence from which the antibody was originally derived). Furthermore, the antibodies of the present disclosure may contain any combination of two or more germline mutations within the framework and/or CDR regions, *e*.*g*., wherein certain individual residues are mutated to the corresponding residue of a particular germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc. The use of antibodies and antigen-binding fragments obtained in this general manner are encompassed within the present disclosure.

The present disclosure also includes methods involving the use of anti-ANGPTL3 antibodies comprising variants of any of the HCVR, LCVR, and/or CDR amino acid sequences disclosed herein having one or more conservative substitutions. For example, the present disclosure includes the use of anti-ANGPTL3 antibodies having HCVR, LCVR, and/or CDR amino acid sequences with, *e*.*g*., 10 or fewer, 8 or fewer, 6 or fewer, 4 or fewer, etc. conservative amino acid substitutions relative to any of the HCVR, LCVR, and/or CDR amino acid sequences disclosed herein.

The term "surface plasmon resonance", as used herein, refers to an optical phenomenon that allows for the analysis of real-time interactions by detection of alterations in protein concentrations within a biosensor matrix, for example using the BIAcore^{™} system (Biacore Life Sciences division of GE Healthcare, Piscataway, NJ).

The term "K_{D} ", as used herein, is intended to refer to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

According to certain aspects , the anti-ANGPTL3 antibodies used in the methods of the present disclosure are antibodies with pH-dependent binding characteristics. As used herein, the expression "pH-dependent binding" means that the antibody or antigen-binding fragment thereof exhibits "reduced binding to ANGPTL3 at acidic pH as compared to neutral pH" (for purposes of the present disclosure, both expressions may be used interchangeably). For the example, antibodies "with pH-dependent binding characteristics" includes antibodies and antigen-binding fragments thereof that bind to ANGPTL3 with higher affinity at neutral pH than at acidic pH. In certain aspects, the antibodies and antigen-binding fragments of the present disclosure bind ANGPTL3 with at least 3, 5, 10, 15, 20, 25, 30, 35, 40, 45, 50, 55, 60, 65, 70, 75, 80, 85, 90, 95, 100, or more times higher affinity at neutral pH than at acidic pH.

According to this aspect of the disclosure, the anti-ANGPTL3 antibodies with pH-dependent binding characteristics may possess one or more amino acid variations relative to the parental anti-ANGPTL3 antibody. For example, an anti-ANGPTL3 antibody with pH-dependent binding characteristics may contain one or more histidine substitutions or insertions, *e*.*g*., in one or more CDRs of a parental anti-ANGPTL3 antibody. Thus, according to certain aspects of the disclosure, methods are provided comprising administering an anti-ANGPTL3 antibody which comprises CDR amino acid sequences (*e*.*g*., heavy and light chain CDRs) which are identical to the CDR amino acid sequences of a parental ANGPTL3 antibody except for the substitution of one or more amino acids of one or more CDRs of the parental antibody with a histidine residue. The anti-ANGPTL3 antibodies with pH-dependent binding may possess, *e*.*g*., 1, 2, 3, 4, 5, 6, 7, 8, 9, or more histidine substitutions, either within a single CDR of a parental antibody or distributed throughout multiple (*e*.*g*., 2, 3, 4, 5, or 6) CDRs of a parental anti-ANGPTL3 antibody. For example, the present disclosure includes the use of anti-ANGPTL3 antibodies with pH-dependent binding comprising one or more histidine substitutions in HCDR1, one or more histidine substitutions in HCDR2, one or more histidine substitutions in HCDR3, one or more histidine substitutions in LCDR1, one or more histidine substitutions in LCDR2, and/or one or more histidine substitutions in LCDR3, of a parental anti-ANGPTL3 antibody.

As used herein, the expression "acidic pH" means a pH of 6.0 or less (*e*.*g*., less than about 6.0, less than about 5.5, less than about 5.0, etc.). The expression "acidic pH" includes pH values of about 6.0, 5.95, 5.90, 5.85, 5.8, 5.75, 5.7, 5.65, 5.6, 5.55, 5.5, 5.45, 5.4, 5.35, 5.3, 5.25, 5.2, 5.15, 5.1, 5.05, 5.0, or less. As used herein, the expression "neutral pH" means a pH of about 7.0 to about 7.4. The expression "neutral pH" includes pH values of about 7.0, 7.05, 7.1, 7.15, 7.2, 7.25, 7.3, 7.35, and 7.4.

In the present invention, the inhibitor of ANGPTL3 is evinacumab.

### Preparation of Human Antibodies

Anti-ANGPTL3 antibodies can be made according to any method of antibody production/isolation known in the art. For example, antibodies for use in the methods of the present disclosure may be made by hybridoma technologies, by phage display, by yeast display, etc. Antibodies for use in the methods of the present disclosure may be, *e*.*g*., chimeric antibodies, humanized antibodies, or fully human antibodies.

Methods for generating human antibodies in transgenic mice are known in the art. Any such known methods can be used in the context of the present disclosure to make human antibodies that specifically bind ANGPTL3.

For example, using VELOCIMMUNE^{™} technology (see, for example, US 6,596,541, Regeneron Pharmaceuticals) or any other known method for generating monoclonal antibodies, high affinity chimeric antibodies to ANGPTL3 are initially isolated having a human variable region and a mouse constant region. The VELOCIMMUNE^{®} technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody.

Generally, a VELOCIMMUNE^{®} mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. The antibodies are characterized and selected for desirable characteristics, including affinity, selectivity, epitope, etc., using standard procedures known to those skilled in the art. The mouse constant regions are replaced with a desired human constant region to generate the fully human antibody of the invention, for example wild-type or modified IgG1 or IgG4. While the constant region selected may vary according to specific use, high affinity antigen-binding and target specificity characteristics reside in the variable region.

In general, the antibodies that can be used in the methods of the present disclosure possess high affinities, as described above, when measured by binding to antigen either immobilized on solid phase or in solution phase. The mouse constant regions are replaced with desired human constant regions to generate the fully human antibodies of the disclosure. While the constant region selected may vary according to specific use, high affinity antigen-binding and target specificity characteristics reside in the variable region.

Specific examples of human antibodies or antigen-binding fragments of antibodies that specifically bind ANGPTL3, which can be used in the context of the methods of the present disclosure include antibodies or antigen-binding proteins comprising the six CDRs (HCDR1, HCDR2, HCDR3, LCDR1, LCDR2 and LCDR3) from the heavy and light chain variable region (HCVR/LCVR) amino acid sequence pair comprising SEQ ID NOs: 1/5.

In certain aspects of the disclosure, the anti-ANGPTL3 antibody, or antigen-binding fragment thereof, that can be used in the methods comprises heavy and light chain complementarity determining regions (HCDR1-HCDR2-HCDR3/LCDR1-LCDR2-LCDR3) comprising the amino acid sequences of SEQ ID NOs:2, 3, 4, 6, 7 and 8.

In certain aspects of the disclosure, the anti-ANGPTL3 antibody, or antigen-binding fragment thereof, that can be used in the methods comprises an HCVR having the amino acid sequence of SEQ ID NO:1 and an LCVR having the amino acid sequence of SEQ ID NO:5.

### Pharmaceutical Compositions and Methods of Administration

The present invention includes administering an ANGPTL3 inhibitor to a patient in combination with a statin, an inhibitor of cholesterol absorption and an inhibitor of microsomal triglyceride transfer protein, wherein the ANGPTL3 inhibitor and the additional agents are contained within the same, or in different pharmaceutical compositions. The pharmaceutical compositions of the invention are formulated with suitable carriers, excipients, and other agents that provide suitable transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN^{™}), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

Exemplary pharmaceutical formulations comprising anti-ANGPTL3 antibodies that can be used in the context of the present invention include any of the formulations as set forth in US 8,795,669 (describing, *inter alia,* exemplary formulations comprising alirocumab), or in WO2013/166448, or WO2012/168491.

Various delivery systems are known and can be used to administer the pharmaceutical composition of the invention, *e*.*g*., encapsulation in liposomes, microparticles, microcapsules, recombinant cells capable of expressing the mutant viruses, receptor mediated endocytosis (see, e.g., Wu et al., 1987, J. Biol. Chem. 262:4429-4432). Methods of administration include, but are not limited to, intradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents.

A pharmaceutical composition of the present invention can be delivered subcutaneously or intravenously with a standard needle and syringe. In addition, with respect to subcutaneous delivery, a pen delivery device readily has applications in delivering a pharmaceutical composition of the present invention. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition of the present invention. Examples include, but are not limited to AUTOPEN^{™} (Owen Mumford, Inc., Woodstock, UK), DISETRONIC^{™} pen (Disetronic Medical Systems, Bergdorf, Switzerland), HUMALOG MIX 75/25^{™} pen, HUMALOG^{™} pen, HUMALIN 70/30^{™} pen (Eli Lilly and Co., Indianapolis, IN), NOVOPEN^{™} I, II and III (Novo Nordisk, Copenhagen, Denmark), NOVOPEN JUNIOR^{™} (Novo Nordisk, Copenhagen, Denmark), BD^{™} pen (Becton Dickinson, Franklin Lakes, NJ), OPTIPEN^{™}, OPTIPEN PRO^{™}, OPTIPEN STARLET^{™}, and OPTICLIK^{™} (Sanofi-Aventis, Frankfurt, Germany), to name only a few. Examples of disposable pen delivery devices having applications in subcutaneous delivery of a pharmaceutical composition of the present invention include, but are not limited to the SOLOSTAR^{™} pen (Sanofi-Aventis), the FLEXPEN^{™} (Novo Nordisk), and the KWIKPEN^{™} (Eli Lilly), the SURECLICK^{™} Autoinjector (Amgen, Thousand Oaks, CA), the PENLET^{™} (Haselmeier, Stuttgart, Germany), the EPIPEN (Dey, L.P.), and the HUMIRA^{™} Pen (Abbott Labs, Abbott Park IL), to name only a few.

In certain situations, the pharmaceutical composition can be delivered in a controlled release system. In one embodiment, a pump may be used (see Langer, supra; Sefton, 1987, CRC Crit. Ref. Biomed. Eng. 14:201). In another embodiment, polymeric materials can be used; see, Medical Applications of Controlled Release, Langer and Wise (eds.), 1974, CRC Pres., Boca Raton, Florida. In yet another embodiment, a controlled release system can be placed in proximity of the compositions target, thus requiring only a fraction of the systemic dose (see, *e*.*g*., Goodson, 1984, in Medical Applications of Controlled Release, supra, vol. 2, pp. 115-138). Other controlled release systems are discussed in the review by Langer, 1990, Science 249:1527-1533.

The injectable preparations may include dosage forms for intravenous, subcutaneous, intracutaneous and intramuscular injections, drip infusions, etc. These injectable preparations may be prepared by known methods. For example, the injectable preparations may be prepared, *e*.*g*., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc.

### Dosage

The amount of an ANGPTL3 inhibitor administered to a subject according to the methods of the present invention is, generally, a therapeutically effective amount. As used herein, the phrase "therapeutically effective amount of an ANGPTL3 inhibitor" means a dose of an ANGPTL3 inhibitor, when administered in combination with a statin, ezetimibe and lomitapide, results in a detectable reduction (at least about 5%, 10%, 15%, 20%, 25%, 30%, 35%, 40%, 45%, 50%, 55%, 60%, 65%, 70%, 75%, or more from baseline) in one or more parameters selected from the group consisting of total cholesterol, LDL-C, ApoB, ApoA-1, Apo CIII, non-HDL-C, VLDL-C, triglycerides, and Lp(a), or an amount that reduces or eliminates a patient's need for other therapeutic agents, or interventions, such as, for example, lipoprotein apheresis.

The amount of ANGPTL3 inhibitor administered to a subject according to the present invention is, generally, a therapeutically effective amount. As used herein, the phrase "therapeutically effective amount of an ANGPTL3 inhibitor" means a dose of ANGPTL3 inhibitor, when combined with the therapeutic agents described herein, results in a detectable reduction (at least about 5%, 10%, 15%, 20%, 25%, 30%, 35%, 40%, 45%, 50%, 55%, 60%, 65%, 70%, 75%, or more from baseline) in one or more parameters selected from the group consisting of total cholesterol, LDL-C, ApoB, ApoA-1, Apo CIII, non-HDL-C, VLDL-C, triglycerides, and Lp(a).

In the case of an anti-ANGPTL3 antibody, a therapeutically effective amount can be from about 0.05 mg to about 600 mg, *e*.*g*., about 0.05 mg, about 0.1 mg, about 1.0 mg, about 1.5 mg, about 2.0 mg, about 10 mg, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, or about 600 mg, of the anti-ANGPTL3 antibody.

The amount of anti-ANGPTL3 antibody contained within the individual doses may be expressed in terms of milligrams of antibody per kilogram of patient body weight (*i.e.,* mg/kg). For example, the anti-ANGPTL3 antibody may be administered to a patient at a dose of about 0.0001 to about 20 mg/kg of patient body weight.

### Combination Therapies

The present invention may also comprise administering an ANGPTL3 inhibitor in combination with a statin, ezetimibe and lomitapide to a patient who is non-responsive to, inadequately controlled by, or intolerant to other standard lipid lowering therapies. In certain embodiments, the need for further administration of the standard lipid lowering therapy may be eliminated altogether. In certain embodiments, the combined use of the ANGPTL3 inhibitor with the other agents described herein may be used in combination with ("on top of") the patient's previously prescribed lipid lowering therapy. For example, in the context of lowering at least one lipid/lipoprotein parameter in a patient suffering from hyperlipidemia (*e*.*g*. hypercholesterolemia), wherein the patient is non-responsive to, inadequately controlled by, or intolerant to a standard lipid lowering therapy, a combination of an ANGPTL3 inhibitor with ezetimibe and lomitapide may be administered to a patient in combination with a stable daily therapeutic statin regimen. Exemplary daily therapeutic statin regimens that may be used in the context of the present invention, include, *e*.*g*., atorvastatin (10, 20, 40 or 80 mg daily), (atorvastatin/ezetimibe 10/10 or 40/10 mg daily), rosuvastatin (5, 10 or 20 mg daily), cerivastatin (0.4 or 0.8 mg daily), pitavastatin (1, 2 or 4 mg daily), fluvastatin (20, 40 or 80 mg daily), simvastatin (5, 10, 20, 40 or 80 mg daily), simvastatin/ezetimibe (10/10, 20/10, 40/10 or 80/10 mg daily), lovastatin (10, 20, 40 or 80 mg daily), pravastatin (10, 20, 40 or 80 mg daily), and combinations thereof. Other lipid modifying therapies that an ANGPTL3 inhibitor may be administered in combination with in the context of the present invention include, *e*.*g*., (1) an agent which increase lipoprotein catabolism (such as niacin); and/or (2) activators of the LXR transcription factor that plays a role in cholesterol elimination such as 22-hydroxycholesterol.

### Administration Regimens

According to certain embodiments of the present invention, multiple doses of an ANGPTL3 inhibitor *(i.e.,* a pharmaceutical composition comprising an ANGPTL3 inhibitor) may be administered to a subject over a defined time course (*e*.*g*., on top of a daily therapeutic statin regimen or other background lipid modifying therapy), in addition to administration of ezetimibe and lomitapide. The methods according to this aspect of the invention comprise sequentially administering to a subject multiple doses of an ANGPTL3 inhibitor. As used herein, "sequentially administering" means that each dose of ANGPTL3 inhibitor is administered to the subject at a different point in time, *e*.*g*., on different days separated by a predetermined interval (*e*.*g*., hours, days, weeks or months). The present invention includes sequentially administering to the patient a single initial dose of an ANGPTL3 inhibitor, followed by one or more secondary doses of the ANGPTL3 inhibitor, and optionally followed by one or more tertiary doses of the ANGPTL3 inhibitor.

The terms "initial dose," "secondary doses," and "tertiary doses," refer to the temporal sequence of administration of the individual doses of a pharmaceutical composition comprising a ANGPTL3 inhibitor. Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of the ANGPTL3 inhibitor, but generally may differ from one another in terms of frequency of administration. In certain embodiments, however, the amount of the ANGPTL3 inhibitor contained in the initial, secondary and/or tertiary doses varies from one another (*e*.*g*., adjusted up or down as appropriate) during the course of treatment. In certain embodiments, two or more (*e*.*g*., 2, 3, 4, or 5) doses are administered at the beginning of the treatment regimen as "loading doses" followed by subsequent doses that are administered on a less frequent basis (*e*.*g*., "maintenance doses").

According to exemplary embodiments of the present invention, each secondary and/or tertiary dose is administered 1 to 26 (e.g., 1, 1½, 2, 2½, 3, 3½, 4, 4½, 5, 5½, 6, 6½, 7, 7½, 8, 8½, 9, 9½, 10, 10½, 11, 11½, 12, 12½, 13, 13½, 14, 14½, 15, 15½, 16, 16½, 17, 17½, 18, 18½, 19, 19½, 20, 20½, 21, 21½, 22, 22½, 23, 23½, 24, 24½, 25, 25½, 26, 26½, or more) weeks after the immediately preceding dose. The phrase "the immediately preceding dose," as used herein, means, in a sequence of multiple administrations, the dose of antigen-binding molecule, which is administered to a patient prior to the administration of the very next dose in the sequence with no intervening doses.

The methods according to this aspect of the invention may comprise administering to a patient any number of secondary and/or tertiary doses of an ANGPTL3 inhibitor. For example, in certain embodiments, only a single secondary dose is administered to the patient. In other embodiments, two or more (*e*.*g*., 2, 3, 4, 5, 6, 7, 8, or more) secondary doses are administered to the patient. Likewise, in certain embodiments, only a single tertiary dose is administered to the patient. In other embodiments, two or more (*e*.*g*., 2, 3, 4, 5, 6, 7, 8, or more) tertiary doses are administered to the patient.

In embodiments involving multiple secondary doses, each secondary dose may be administered at the same frequency as the other secondary doses. For example, each secondary dose may be administered to the patient 1 to 2, 4, 6, 8 or more weeks after the immediately preceding dose. Similarly, in embodiments involving multiple tertiary doses, each tertiary dose may be administered at the same frequency as the other tertiary doses. For example, each tertiary dose may be administered to the patient 1 to 2, 4, 6, 8 or more weeks after the immediately preceding dose. Alternatively, the frequency at which the secondary and/or tertiary doses are administered to a patient can vary over the course of the treatment regimen. The frequency of administration may also be adjusted during the course of treatment by a physician depending on the needs of the individual patient following clinical examination. Likewise, the doses of the concomitant therapies, e.g. the statin, ezetimibe and lomitapide may be adjusted during the course of treatment by a physician according to the normalization of lipid levels observed during the course of treatment.

### EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1. Generation of Human Antibodies to Human ANGPTL3

The exemplary ANGPTL3 antibody used in the following Example is the human anti-ANGPTL3 antibody known as "evinacumab." Evinacumab has the following amino acid sequence characteristics: a heavy chain variable region (HCVR) comprising SEQ ID NO:1 and a light chain variable domain (LCVR) comprising SEQ ID NO:5; a heavy chain complementarity determining region 1 (HCDR1) comprising SEQ ID NO:2, a HCDR2 comprising SEQ ID NO:3, a HCDR3 comprising SEQ ID NO:4, a light chain complementarity determining region 1 (LCDR1) comprising SEQ ID NO:6, a LCDR2 comprising SEQ ID NO:7 and a LCDR3 comprising SEQ ID NO:8.

### Example 2: Safety and Efficacy of Evinacumab, a Monoclonal Antibody to ANGPTL3 in Patients with Homozygous Familial Hypercholesterolemia Receiving Concomitant Lipid-lowering Therapies

Homozygous familial hypercholesterolemia (HoFH) involves profound genetic deficiencies in the low-density lipoprotein (LDL) receptor pathway, leading to catastrophically elevated LDL-cholesterol (LDL-C) and severe premature atherosclerosis; responses to statins and PCSK9 antibodies are limited. Preclinical studies and human genetic analyses suggest that inhibition of angiopoietin-like protein (ANGPTL3) lowers LDL-C and provides cardiovascular benefit, independently of the LDL receptor. Evinacumab, a human ANGPTL3 antibody, was administered to nine HoFH adults (three null homozygotes) already on maximally-tolerated conventional therapies. LDL-C decreased 49% (range -25% to -90%) at week 4 (primary endpoint). Overall mean peak reduction in LDL-C was -58±18% (-90% to -33%) between weeks 4 and 12, showing that ANGPTL3 inhibition by evinacumab substantially reduces LDL-C in HoFH patients.

Low-density lipoproteins (LDL) play a major role in the initiation and progression of atherosclerosis and risk of cardiovascular disease. Familial hypercholesterolemia (FH) is typically a disorder occurring through mutations in genes encoding proteins that regulate clearance of LDL. These include the genes for the low-density lipoprotein receptor (*LDLR*)*,* apolipoprotein B (APOB), proprotein convertase subtilisin/kexin type 9 *(PCSK9),* and low-density lipoprotein receptor adaptor protein 1 (*LDLRAP1*) (Cuchel, et al. 2014 Eur Heart J 35:2146-57). Patients with heterozygous FH usually have untreated plasma LDL-cholesterol (LDL-C) levels ranging from 350 to 550 mg per deciliter and generally respond to lipid-lowering therapies, including moderate to high doses of high-potency statins, ezetimibe, and PCSK9 antibodies (Goldberg, et al. 2011 J Clin Lipidol 2011;5:S1-8; Kastelein, et al. 2014 Cardiovasc Drugs Ther 28:281-9). Homozygous FH (HoFH) is a rare disease, which affects 1 in 160,000 to 300,000 people. Patients with HoFH carry two FH-causing mutations (homozygous or compound heterozygous), have much higher untreated LDL-C levels, generally ranging from 500 to 1000 mg per deciliter (Kolansky, et al. 2008 The American journal of cardiology 102:1438-43), and are significantly less responsive or unresponsive to standard lipid-lowering therapies. Most individuals with HoFH develop severe xanthomatosis, coronary heart disease, and peripheral atherosclerosis at an early age, and can die before the age of 30 if left untreated (Nordestgaard, et al. 2013 Eur Heart J 34:3478-90a).

Genetic and phenotypic heterogeneity in HoFH can translate into broad variability in cardiovascular disease manifestation and response to lipid-lowering therapies. Some FH-causing mutations result in defective LDL receptors with residual activity, whereas others have no activity and therefore do not respond to conventional lipid-lowering medications, such as statins and PCSK9 antibodies, which mainly target the process of LDL-receptor expression (Santos PC, Pereira AC. 2015 Pharmacogenomics 16:1743-50; Rader DJ, Kastelein JJ. 2014 Circulation 129:1022-32). Drugs with mechanisms of action unrelated to the LDL receptor, such as lomitapide and mipomersen, have been approved recently for treating HoFH, but their use can be hampered by tolerability and safety issues.

Angiopoietin-like protein 3 (ANGPTL3) is a secreted protein expressed in the liver. It acts to increase plasma levels of triglycerides, LDL-C, and high-density lipoprotein cholesterol (HDL-C) by inhibiting the activity of lipoprotein lipase and endothelial lipase or by modulating the clearance of triglyceride-rich lipoproteins upstream of LDL production (Wang, et al. 2015 J Lipid Res 56:1296-307; Musunuru, et al. 2010 N Engl J Med 363:2220-7). Preclinical studies show that knockout of ANGPTL3, or blockade by an antibody, can lower triglycerides and LDL-C independently of the LDL-R, and have benefit in models of atherosclerosis (Ando, et al. 2003 J Lipid Res 44:1216-23; Dewey, et al. 2017 New Engl J Med*;* in press). Consistent with this, large-scale genetic studies in man show that loss-of-function mutations in *ANGPTL3* lead to reduced plasma levels of triglycerides, LDL-C, and HDL-C (Robciuc, et al. 2013 Arteriosclerosis, thrombosis, and vascular biology 33:1706-13; Pisciotta, et al. 2012 Circ Cardiovasc Genet 5:42-50; Minicocci, et al. 2013 J Lipid Res 54:3481-90; Wang, et al. 2015 Proc Natl Acad Sci U S A 112:11630-5; Noto, et al. 2012 Arteriosclerosis, thrombosis, and vascular biology 32:805-9; Martin-Campos, et al. 2012 Clinica chimica acta; international journal of clinical chemistry 413:552-5), and even more importantly, that these lipid changes associated with *ANGPTL3* mutations are also associated with protection from cardiovascular disease (Stitziel, et al. 2017 Journal of the American College of Cardiology)*.* Altogether, the preclinical studies as well as the human genetic analyses suggest that ANGPTL3-inhibiting therapies could reduce LDL-C and provide benefit in patients with FH, including those suffering from profound homozygous disease, Evinacumab is a fully human monoclonal antibody that specifically blocks ANGPTL3 (Gusarova, et al. 2015 J Lipid Res 56:1308-17). In normal healthy volunteers, evinacumab was well tolerated and reduced the three major lipid fractions. A phase 2 study was conducted to determine whether evinacumab reduced LDL-C levels in nine patients with genetically and phenotypically confirmed HoFH, including patients homozygous for null mutations completely lacking LDLR activity.

### METHODS

Patients: The nine patients (5 men, 4 women) were selected based on their genotypes and phenotypes. All presented a history of LDL-C >500 mg per deciliter or >400 mg per deciliter after portacaval shunt, premature atherosclerosis (8 of 9 with prior history of cardiovascular events) and severe xanthomatosis, and were homozygotes or compound heterozygotes for known FH-causing *LDLR* mutations (Hobbs, et al. 1992 Hum Mutat 1:445-66). Three patients were null homozygotes. All patients were on maximally tolerated lipid-lowering therapy.

Study Treatment: The patients were required to maintain their usual background lipid-lowering therapy and diet and exercise regimens throughout the study. All patients received a single open-label dose of evinacumab 250 mg subcutaneously in the abdominal area during the baseline visit and a single 15 mg per kilogram intravenous dose of evinacumab 2 weeks later. The sterile, lyophilized evinacumab drug product was supplied in a 5-ml single-use glass vial for reconstitution to a concentration of 100 mg per milliliter for subcutaneous doses and 50 mg per milliliter for intravenous doses. Patients were followed for a period of up to 24 weeks after the intravenous dose to allow for washout of evinacumab, and were offered enrollment in an extension study.

Pharmacodynamic Assessment: Fasting blood samples were collected before administration of study drug, at baseline and at regular intervals during the open-label treatment period and safety follow-up period, for measurement of LDL-C, non-HDL-C, total cholesterol, HDL-C, apolipoprotein B, lipoprotein(a), triglycerides, apolipoprotein A-1, and other parameters. The primary endpoint was the mean ± standard deviation (SD) percent change in LDL-C from baseline to week 4.

### RESULTS

Even though most patients were on maximally tolerated therapies, mean±SD baseline LDL-C was 376.0±240.9 milligrams per deciliter (mg/dL); one patient who had failed statin therapy, and was removed from weekly apheresis, had a baseline LDL-C of 756 mg/dL. All nine patients reported the occurrence of at least one adverse event, but none led to treatment discontinuation. One event (coronary artery disease due to underlying disease) was serious but was not considered as related to the study medication. Six events were considered as related to the study medication, two of which were injection site reactions of mild severity, one was myalgia of moderate severity, and one was epistaxis of severe severity.

Drug Response: The mean±SD percent change in LDL-C from baseline to week 4 (prespecified primary endpoint) following evinacumab administration was -49±23% (range: -90% to -25%), with an absolute change from baseline of -157±90 (range: -323 to -71) mg per deciliter (Table 1, below). The mean±SD achieved LDL-C value at week 4 was 219±191 mg per deciliter. Over the same period, percent change in apolipoprotein B decreased by 46±18% (Table 2, below), non-HDL-C by 49±22% (Table 3, below), triglycerides by 47% (median, interquartile range -57% to -38%), and HDL-C by 36±16%. Overall mean±SD peak reduction in LDL-C occurring between weeks 4 and 12 was -58±18% (range -90% to -33%), with an absolute peak reduction in LDL-C of 202 mg/dL. At week 4 (2 weeks after the intravenous dose), one patient achieved a reduction in LDL-C greater than 80%. In the 3 homozygous null patients, the mean±SD peak reduction in LDL-C through week 12 was -48±13% (range -60% to -33%).

**Table 1: Effect of AngPTL3 Inhibition on Plasma LDL-C Concentrations**

| LDL-C | |
|---|---|
| Visit | Mean of Percent Change from Baseline ± SE (%) |
| BL (wk 0) | 0 |
| Day 4 | -12.8 ± 3.7 |
| Week 1 | -24 ± 7.0 |
| Week 2 | -30.2 ± 8.1 |
| Week 3 | -41.4 ± 8.3 |
| Week 4 | -49.2 ± 7.7 |
| Week 5 | -46.8 ± 5.1 |
| Week 6 | -52.1 ± 4.9 |
| Week 8 | -51.6 ± 6.0 |
| Week 10 | -45.6 ± 4.6 |
| Week 12 | -36.6 ± 6.4 |

**Table 2: Effect of AngPTL3 Inhibition on Apolipoprotein B Concentrations**

| Apolipoprotein B | |
|---|---|
| Visit | Mean of Percent Change from Baseline ± SE (%) |
| BL (wk 0) | 0 |
| Week 2 | -24.2 ± 7 |
| Week 3 | -38.6 ± 7.3 |
| Week 4 | -45.9 ± 6.1 |
| Week 5 | -42.3 ± 4.7 |
| Week 6 | -43.1 ± 4.9 |
| Week 8 | -42.7 ± 4.8 |
| Week 12 | -29.5 ± 7.2 |

**Table 3: Effect of AngPTL3 Inhibition on Non-HDL-C Concentrations**

| Non-HDL-C | |
|---|---|
| Visit | Mean of Percent Change from Baseline ± SE (%) |
| BL (wk 0) | 0 |
| Day 4 | -13.6 ± 3.6 |
| Week 1 | -24.1 ± 6.7 |
| Week 2 | -29.6 ± 7.8 |
| Week 3 | -41.6 ± 8 |
| Week 4 | -48.9 ± 7.4 |
| Week 5 | -46.6 ± 5.0 |
| Week 6 | -51.5 ± 4.8 |
| Week 8 | -50.6 ± 5.7 |
| Week 10 | -44.8 ± 4.4 |
| Week 12 | -36.4 ± 6.2 |

Administration of the fully human monoclonal ANGPTL3-blocking antibody evinacumab in nine adults with HoFH, including three null homozygotes, resulted in meaningful reductions in LDL-C. Importantly, these reductions were on top of baseline levels already achieved on stable, maximally-tolerated lipid-lowering therapy with or without lomitapide, PCSK9 monoclonal antibodies or portacaval shunt. These results provide proof-of-concept of a substantial additional reduction in LDL-C by evinacumab, on top of standard of care, in the treatment of HoFH, with the potential for LDL-C normalization in some patients presenting with extremely high LDL-C levels. Evinacumab given as a 250-mg subcutaneous injection at baseline and as a 15 mg per kilogram intravenous infusion at week 2 was well tolerated. In a recently reported first-in-human study of healthy human volunteers, evinacumab was also shown to reduce LDL-C and was also well tolerated in a larger number of patients (Dewey, et al. 2017 New Engl J Med; in press). All nine patients, including the three homozygous null patients lacking LDLR activity, demonstrated clinically meaningful reductions in LDL-C from baseline. Together with recent preclinical studies and human genetic analyses, the results suggest that Angptl3 inhibition can not only lower LDL-C and triglycerides, but also provide protection from cardiovascular disease. These studies provide real hope for a well-tolerated and impactful treatment for patients suffering from profound familial hypercholesterolemia. Based on the Cholesterol Treatment Trialists' Collaboration (CTTC) analyses (Cholesterol Treatment Trialists (CTT) Collaboration 2010 Lancet 376:1670-81), an absolute decrease of 39 mg/dL in LDL-C corresponds to a 22% relative risk reduction over 4-5 years of statin treatment; recent outcomes data with a PCSK9 antibody support similar risk reductions per unit LDL-C reduction when accounting for shorter treatment duration (Sabatine, et al. 2017 N Engl J Med 3-17-2017 e-publication; in press). Together with the genetic data on the protective effects of ANGPTL3 mutations on cardiovascular risk (Stitziel, et al. 2017 Journal of the American College of Cardiology 69(16):2054-2063), the absolute reductions of a 150-200 mg/dL achieved with evinacumab in HoFH patients could have unprecedented benefit for these very high risk patients.

The mechanism that led to such large reductions in LDL-C is currently under investigation. Evinacumab relieves the normal inhibition, by ANGPTL3, of both lipoprotein lipase (a major regulator of triglycerides and endothelial lipase (a regulator of HDL-C (Shimamura, et al. 2007 Arteriosclerosis, thrombosis, and vascular biology 27:366-72); thus, the lowering of both triglycerides and HDL-C by evinacumab. The instant results, combined with the results of a recent *in vivo* study (Wang, et al. 2015 J Lipid Res 56:1296-307), suggest that the effects of evinacumab on LDL-C involve a combination of canonical and noncanonical mechanisms acting upstream of LDL-particle formation. In a mouse model, inactivation of ANGPTL3 with evinacumab did not affect the number of VLDLs secreted by the liver, but qualitatively altered the VLDL particles that were made. Following secretion, VLDLs are rapidly hydrolyzed to form triglyceride-poorer VLDL remnants due to evinacumab-induced up-regulation of lipoprotein lipase, which may increase their clearance through receptors other than LDL receptors. In terms of the modest reductions seen with HDL-C, extensive previous genetic analyses involving endothelial lipase (Voight, et al. 2012 Lancet 380:572-80), as well as the recent genetic findings with ANGPTL3 protection (Dewey 2017, Stitziel 2017), are consistent with the emerging view that levels of HDL-C do not directly affect cardiovascular risk (Ko, et al. 2016 Journal of the American College of Cardiology 68:2073-83).

Patient C and G concomitantly received lomitapide-a microsomal triglyceride transfer protein inhibitor-during the study. These patients showed a 90% and 44% reduction in LDL-C, respectively, 2 weeks after evinacumab intravenous administration, raising the hypothesis of a multiplicative synergy between lomitapide (which affects VLDL production) and evinacumab (which affects the characteristics of secreted VLDL). However, patient D did not receive lomitapide and showed a- 77% reduction in LDL-C at week 4.

The results reported herein provide proof-of-concept that ANGPTL3 inhibition with evinacumab leads to a substantial additional reduction in LDL-C in patients with HoFH on stable lipid-lowering therapy, including those who have null/null mutations. Evinacumab add-on therapy allowed normalization of LDL-C concentrations in four HoFH participants in this study. For example, Patient C, a 47-year-old woman, presented LDL-C values above 800 mg per deciliter at age 26. Her lipid profile progressively improved (reaching 150 to 170 mg per deciliter) with the successive introduction of high-dose statins, ezetimibe and lomitapide. LDL-C reached 15 mg per deciliter at week 4, 2 weeks after evinacumab intravenous administration.

### Example 3: Inhibition of ANGPTL3 by evinacumab reduced triglycerides (TGs) and LDL-C in subjects presenting with modest elevations in TGs and/or LDL-C

Elevations in LDL-C and TGs have been linked to increased risk in CHD. Recent discoveries have demonstrated a central role for Angiopoietin like - 3 (ANGPTL3) in lipid metabolism. Loss of function (LoF) of ANGPTL3 in humans has been associated with reductions in TGs, LDL-C, and HDL-C. Evinacumab is a human monoclonal antibody specific for ANGPTL3 that is being developed for treatment of dyslipidemia, including hypertriglyceridemia and hypercholesterolemia.

Methods: The instant study constituted a phase 1, first-in-human, ascending single-dose, placebo (PBO)-controlled, double-blind study of evinacumab administered subcutaneously (SC) or intravenously (IV) in subjects with elevations of TGs (150≤ TG ≤450 mg/dL) and/or LDL C (≥100 mg/dL). Eighty-three subjects were randomized into the study (9 in PBO SC; 12 in PBO IV; 11 in 75 mg SC: 12 in 150 mg SC, 9 in 250 mg SC, 10 in 5 mg/kg IV, 9 in 10 mg/kg IV, and 11 in 20 mg/kg IV).

Results: Evinacumab was shown to be well tolerated in this trial. Forty-one (41) subjects reported at least one treatment emergent adverse event (TEAE): 32[± 51.6%] in the evinacumab group vs. 9[± 42.9%] in the PBO group. None were serious, and no subject discontinued due to a TEAE. The most frequent TEAEs were headache (7 [11.3%] vs. 0 [0%]) and increases in ALT/AST [>2X ULN] (5 treated subjects vs 1 PBO subject). There was no dose-related safety trend. Maximum TG reductions were observed on Day 4, with a median % change from baseline of -1.0% to -75.0% across the evinacumab doses and +25.3% for PBO. The mean % changes of LDL-C from baseline on Day 11 were -3.4% to -25.5% across the evinacumab doses and +10.2 for PBO. The duration of TG reduction and LDL was dose-dependent and extended to 64 and 43 days, respectively, after 20 mg/kg IV evinacumab administration. Dose-dependent reductions in HDL-C, VLDL-C, total cholesterol, non-HDL-C, ApoA1, and ApoB were also observed, but there were no apparent effects on Lp(a).

Administration of evinacumab in healthy subjects with moderately elevated TGs and/or LDL-C was generally well-tolerated. Furthermore, evinacumab induced rapid and substantial reductions in TGs, as well as reductions in LDL-C and HDL-C, recapitulating the observed hypolipoproteinemia in individuals homozygous for ANGPTL3 LOF mutations.

## Claims

1. An inhibitor of angiopoietin-like protein 3 (ANGPTL3) for use in a method of treating a patient suffering from familial hypercholesterolemia in combination with a statin and two lipid-lowering agents other than a statin, wherein the method comprises administering to the patient a therapeutically effective amount of the statin, the two lipid-lowering agents other than a statin and an inhibitor of ANGPTL3, wherein
- the ANGPTL3 inhibitor is evinacumab;
- the statin is selected from the group consisting of atorvastatin, pitavastatin, lovastatin, simvastatin, pravastatin, fluvastatin and rosuvastatin; and
- the first lipid-lowering agent other than a statin is an agent that inhibits cholesterol absorption.

2. The ANGPTL3 inhibitor for use according to claim 1, wherein the familial hypercholesterolemia is selected from the group consisting of heterozygous familial hypercholesterolemia (HeFH) and homozygous familial hypercholesterolemia (HoFH).

3. The ANGPTL3 inhibitor for use according to claim 1 or 2, wherein:
(a) the statin is atorvastatin administered orally once a day at a dose of about 10 mg to about 80 mg; or
(b) the statin is rosuvastatin administered orally once a day at a dose of about 5 mg to about 40 mg; or
(c) the agent that inhibits cholesterol absorption is ezetimibe, optionally wherein the ezetimibe is administered orally once a day at a dose of about 10 mg.

4. The ANGPTL3 inhibitor for use according to claim 1, wherein the second lipid-lowering agent other than a statin is an agent that inhibits microsomal triglyceride transfer protein (MTTP), optionally wherein:
(a) the agent that inhibits MTTP is lomitapide; or
(b) the agent that inhibits MTTP is lomitapide and the lomitapide is administered orally once a day at a dose of about 5 mg to about 60 mg, optionally wherein the lomitapide is administered orally once a day at a dose of about 20 mg.

5. The ANGPTL3 inhibitor for use according to any one of claims 1-4, wherein:
(a) evinacumab is administered before, during, or after treatment with a statin, ezetimibe, or lomitapide;
(b) evinacumab is administered intravenously at a dose ranging from about 1 mg/kg to about 20 mg/kg of body weight, such as at a dose of about 15 mg/kg of body weight;
(c) evinacumab is administered subcutaneously at a dose ranging from about 50 mg to about 750 mg, such as at a dose ranging from about 250 mg to about 450 mg; or
(d) evinacumab is administered every week, every two weeks, every 3 weeks, every 4 weeks, every 2 months, every 3 months, or every 4 months.

## Patentansprüche

1. Inhibitor des Angiopoietin-ähnlichen Proteins 3 (ANGPTL3) für die Verwendung in einem Verfahren zum Behandeln eines Patienten, der an familiärer Hypercholesterinämie leidet, in Kombination mit einem Statin und zwei anderen lipidsenkenden Mitteln als ein Statin, wobei das Verfahren das Verabreichen einer therapeutisch wirksamen Menge des Statins, der zwei anderer lipidsenkenden Mittel als ein Statin und eines Inhibitors von ANGPTL3 an den Patienten umfasst, wobei
- der ANGPTL3-Inhibitor Evinacumab ist;
- das Statin aus der Gruppe ausgewählt ist, bestehend aus Atorvastatin, Pitavastatin, Lovastatin, Simvastatin, Pravastatin, Fluvastatin und Rosuvastatin; und
- das erste andere lipidsenkende Mittel als ein Statin ein Mittel ist, das die Cholesterinabsorption hemmt.

2. ANGPTL3-Inhibitor für die Verwendung nach Anspruch 1, wobei die familiäre Hypercholesterinämie aus der Gruppe ausgewählt ist, bestehend aus heterozygoter familiärer Hypercholesterinämie (HeFH) und homozygoter familiärer Hypercholesterinämie (HoFH).

3. ANGPTL3-Inhibitor für die Verwendung nach Anspruch 1 oder 2, wobei:
(a) das Statin Atorvastatin ist, das einmal täglich oral in einer Dosis von etwa 10 mg bis etwa 80 mg verabreicht wird; oder
(b) das Statin Rosuvastatin ist, das einmal täglich oral in einer Dosis von etwa 5 mg bis etwa 40 mg verabreicht wird; oder
(c) das Mittel, das die Cholesterinabsorption hemmt, Ezetimib ist, wobei das Ezetimib optional einmal täglich oral in einer Dosis von etwa 10 mg verabreicht wird.

4. ANGPTL3 -Inhibitor für die Verwendung nach Anspruch 1, wobei das zweite andere lipidsenkende Mittel als ein Statin ein Mittel ist, das das mikrosomale Triglycerid-Transferprotein (MTTP) hemmt, wobei optional:
(a) das Mittel, das MTTP hemmt, Lomitapid ist; oder
(b) das Mittel, das MTTP hemmt, Lomitapid ist und das Lomitapid einmal täglich oral in einer Dosis von etwa 5 mg bis etwa 60 mg verabreicht wird, wobei optional das Lomitapid oral einmal täglich in einer Dosis von etwa 20 mg verabreicht wird.

5. ANGPTL3-Inhibitor für die Verwendung nach einem der Ansprüche 1-4, wobei:
(a) Evinacumab vor, während oder nach der Behandlung mit einem Statin, Ezetimib oder Lomitapid verabreicht wird;
(b) Evinacumab intravenös in einer Dosis von etwa 1 mg/kg bis etwa 20 mg/kg Körpergewicht verabreicht wird, wie in einer Dosis von etwa 15 mg/kg Körpergewicht;
(c) Evinacumab subkutan in einer Dosis von etwa 50 mg bis etwa 750 mg verabreicht wird, wie in einer Dosis von etwa 250 mg bis etwa 450 mg; oder
(d) Evinacumab jede Woche, alle zwei Wochen, alle 3 Wochen, alle 4 Wochen, alle 2 Monate, alle 3 Monate oder alle 4 Monate verabreicht wird.

## Revendications

1. Inhibiteur de protéine 3 de type angiopoïétine (ANGPTL3) destiné à une utilisation dans une méthode de traitement d'un patient souffrant d'hypercholestérolémie familiale en association avec une statine et deux agents hypolipidémiants autres qu'une statine, la méthode comprenant l'administration au patient d'une quantité thérapeutiquement efficace de la statine, des deux agents hypolipidémiants autres qu'une statine et d'un inhibiteur d'ANGPTL3 ;
- l'inhibiteur d'ANGPTL3 étant l'évinacumab,
- la statine étant sélectionnée dans le groupe constitué par l'atorvastatine, la pitavastatine, la lovastatine, la simvastatine, la pravastatine, la fluvastatine et la rosuvastatine, et
- le premier agent hypolipidémiant autre qu'une statine est un agent inhibiteur de l'absorption du cholestérol.

2. Inhibiteur d'ANGPTL3 destiné à une utilisation selon la revendication 1, selon laquelle l'hypercholestérolémie familiale est sélectionnée dans le groupe constitué par l'hypercholestérolémie familiale hétérozygote (HeFH) et l'hypercholestérolémie familiale homozygote (HoFH).

3. Inhibiteur d'ANGPTL3 destiné à une utilisation selon la revendication 1 ou 2, selon laquelle :
(a) la statine est l'atorvastatine, administrée par voie orale une fois par jour à une dose d'environ 10 mg à environ 80 mg ; ou
(b) la statine est la rosuvastatine, administrée par voie orale une fois par jour à une dose d'environ 5 mg à environ 40 mg ; ou
(c) l'agent inhibiteur de l'absorption du cholestérol est l'ézétimibe, l'ézétimibe étant éventuellement administré par voie orale une fois par jour à une dose d'environ 10 mg.

4. Inhibiteur d'ANGPTL3 destiné à une utilisation selon la revendication 1, selon laquelle le deuxième agent hypolipidémiant autre qu'une statine est un agent inhibiteur de la protéine microsomale de transfert des triglycérides (MTTP), et selon laquelle éventuellement :
(a) l'agent inhibiteur de la MTTP est le lomitapide ; ou
(b) l'agent inhibiteur de la MTTP est le lomitapide et ce dernier est administré par voie orale une fois par jour à une dose d'environ 5 mg à environ 60 mg, le lomitapide étant éventuellement administré par voie orale une fois par jour à une dose d'environ 20 mg.

5. Inhibiteur d'ANGPTL3 destiné à une utilisation selon l'une quelconque des revendications 1 à 4, selon laquelle :
(a) l'évinacumab est administré avant, pendant ou après un traitement par statine, ézétimibe ou lomitapide ;
(b) l'évinacumab est administré par voie intraveineuse à une dose allant d'environ 1 mg/kg à environ 20 mg/kg de poids corporel, telle qu'une dose d'environ 15 mg/kg de poids corporel ;
(c) l'évinacumab est administré par voie sous-cutanée à une dose allant d'environ 50 mg à environ 750 mg, telle qu'une dose allant d'environ 250 mg à environ 450 mg ; ou
(d) l'évinacumab est administré toutes les semaines, toutes les deux semaines, toutes les 3 semaines, toutes les 4 semaines, tous les 2 mois, tous les 3 mois ou tous les 4 mois.
